# EUROPEAN PATENT APPLICATION

(11) **EP 1 949 876 A1**
(43) Date of publication of application: **30.07.2008**
(21) Application number: 07101206.6
(22) Date of filing: 25.01.2007
(51) Int. Cl.: A61F 6/04

(54) **Condom with expanded glans portion**

(71) Applicant: Ou, Chia-Chen, Lingya District, Kaohsiung City Taiwan, Province of China (CN)
(72) Inventor: Ou, Chia-Chen, Lingya District, Kaohsiung City Taiwan, Province of China (CN)
(74) Representative: Kewitz, Ansgar

(57) **Abstract**

A condom with a structure that allows the user to fully enjoy the natural pleasure in sexual intercourse while hardly feeling the existence of the condom is disclosed. The condom comprises a shaft portion having a diameter substantially the same or slightly smaller than that of the shaft of a male user's penis; a glans portion having a diameter greater than that of the male user's glans; and an amount of lubricant spread over the inner surface of the glans portion. The shaft portion and the glans portion of the condom are connected to each other to form a single piece, which is made of a natural rubber latex. In use, the shaft portion tightly encloses the shaft of the user's penis and the glans portion loosely covers the user's glans, but will be pressed and rubbed against the glans during intercourse.

## Description

### FIELD OF THE INVENTION

The present invention relates to condoms and more particularly to a condom with a structure that allows a user to fully enjoy the natural pleasure in sexual intercourse while hardly feeling the existence of the condom.

### BACKGROUND OF THE INVENTION

The prior art condom is made of a natural rubber latex. In use, the condom covers upon the glans (i.e., glans penis or balanus) of a user. However, the sense of separation brought about by the condom generally deprives its user of a true intimate feeling in sexual intercourse. As a result, men often avoid using condoms, thus risking contracting or spreading venereal diseases, such as AIDS, or causing unwanted pregnancies to their female sexual partners.

Therefore, a condom which allows the user to fully enjoy the natural pleasure in sexual intercourse while hardly feeling the existence of the condom would be beneficial to personal hygiene and public health. This is because men would be more willing to use the condom and therefore the chances of contracting or spreading venereal diseases or causing unwanted pregnancies would be lowered.

### SUMMARY OF THE INVENTION

The primary object of the present invention is to provide a condom with a structure that allows a user to fully enjoy the natural pleasure in sexual intercourse while hardly feeling the existence of the condom.

To achieve the above objects, the condom according to the present invention comprises a shaft portion having a diameter substantially the same or slightly smaller than that of the shaft of a male user's penis; a glans portion having a diameter greater than that of the male user's glans; and an amount of lubricant inside the glans portion. The shaft portion and the glans portion of the condom are connected to each other to form a single piece, which is made of a natural rubber latex. In use, the shaft portion tightly encloses the shaft of the user's penis whereas the glans portion loosely covers the glans with a gap formed between the glans portion of the condom and the glans. When the penis moves forwards and backwards during sexual intercourse, the glans portion will move along with the penis; the glans portion will be pressed and rubbed against the male user's glans and his female partner's vagina to generate a natural pleasant sensation for both.

The various objects and advantages of the present invention will be more readily understood from the following detailed description when read in conjunction with the appended drawings.

### BRIEF DESCRIPTION OF THE DRAWINGS

Fig. 1 shows the structure of a condom according to the present invention enclosing a male user's penis.
Fig. 2A is a schematic view showing a condom according to the present invention in actual use, wherein the glans portion of the condom is pressed and rubbed against the user's glans and the female partner's vagina.

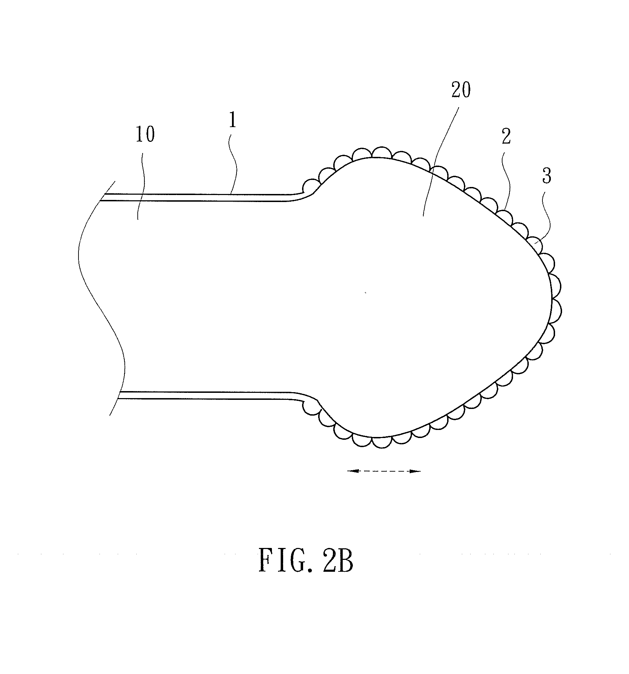

Fig.2B is a magnifying schematic view showing a condom according to the present invention in actual use, wherein the glans portion of the condom is pressed and rubbed against the user's glans and the female partner's vagina.

### DETAILED DESCRIPTION OF THE INVENTION

In order that those skilled in the art can further understand the present invention, a detailed description of the present invention is provided in the following. This description and the appended drawings are only used to help those skilled in the art to understand the objects, features, and characteristics of the present invention, and are not intended to confine the scope of the present invention defined in the appended claims.

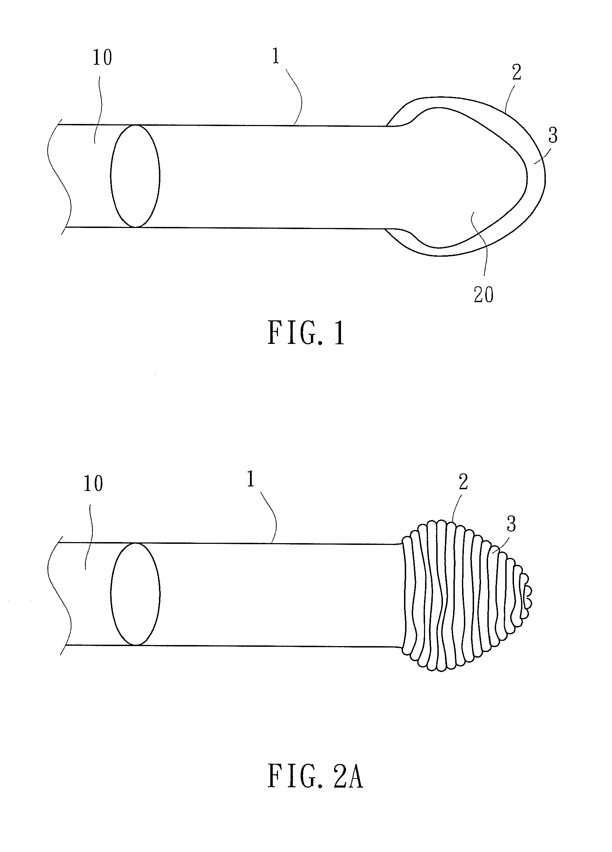

In Fig. 1, the condom 10 of the present invention is illustrated. The condom of the present invention comprises the following elements:
a shaft portion 1, which is a cylindrical shell having an inner diameter substantially the same or slightly smaller than that of the shaft of a male user's penis;
a glans portion 2, which is a substantially spherical shell having a diameter greater than that of the male user's glans and having an opening in communication with one end of the shaft portion 1to form an integral piece with the shaft portion1; and
an amount of lubricant 3 spread over an inner surface of the glans portion 2.

The shaft portion 1 and the glans portion 2 of the condom 10 are made of a natural rubber latex.

In use, the shaft portion 1 tightly encloses the shaft of the penis, preventing the lubricant 3 and sperms from leaking out, and the glans portion 2 loosely covers the glans 20 of the penis with a gap formed between the glans portion 2 of the condom 10 and the glans 20. When the penis moves forwards and backwards during sexual intercourse, the glans portion 2 will move along with the penis and the glans portion 2 with the lubricant 3 spread over the inner surface of the glans portion 2 will be pressed and rubbed against the male user's glans 20 and his female partner's vagina to generate a natural pleasant sensation for both partners.

In summary, in the present invention, the condom according to the present invention comprises a shaft portion 1 with a diameter substantially the same or slightly smaller than that of the shaft of a male user's penis; a glans portion 2 with a diameter greater than that of the male user's glans 20; and an amount of lubricant spread over the inner surface of the glans portion 2. It is known that the shaft of the penis of a man on average has a diameter of about 35 mm and the widest portion of the glans has a diameter of about 40 mm (when the penis is nonerect). The diameters should vary from person to person. The size of the shaft portion 1 and the size of the glans portion 2 of the condom 10 should be selected and adapted according to the general needs of the local population.

The present invention is thus described. It will be obvious that the same may be varied and modified in many ways. Such variations and modifications are not to be regarded as a departure from the spirit and scope of the present invention, and all such variations and modifications as would be obvious to one skilled in the art should be encompassed within the scope of the following claims.

## Claims

1. A condom (10), comprising:
a shaft portion (1), which is a cylindrical shell having an inner diameter substantially the same or slightly smaller than that of the shaft of a user's penis;
a glans portion (2), which is a substantially spherical shell having a diameter greater than that of the glans (20) of the user's penis and having an opening in communication with one end of the shaft portion (1) to form an integral piece with the shaft portion (1); and
a predetermined amount of lubricant (3) spread over an inner surface of the glans portion (2);
wherein the shaft portion (1) and the glans portion (2) are made of a natural rubber latex; and
when in use, the shaft portion (1) tightly encloses the shaft of the user's penis and the glans portion (2) loosely covers the user's glans (20) with a gap formed between the glans portion (1) and the glans (20); when the penis moves forwards and backwards in sexual intercourse, the glans portion (2) will be pressed and rubbed against the glans (20) to generate a natural pleasant sensation.
